# EUROPEAN PATENT SPECIFICATION

(11) **EP 1 897 536 B1**
(45) Date of publication and mention of the grant of the patent: **27.06.2012**
(21) Application number: 07017503.9
(22) Date of filing: 06.09.2007
(51) Int. Cl.: A61K 47/48, C07K 14/705, A61K 9/127, A61K 9/16, A61K 9/51

(54) **Surface cross-linked lipidic particles, methods of production and uses therefor**
Oberflächenvernetzte Lipidteilchen sowie Verfahren zu ihrer Herstellung und Verwendung
Particules lipides réticulées en surface, leurs procédés de production et utilisations

(30) Priority: 07.09.2006 US 842647 P
(43) Date of publication of application: 12.03.2008
(73) Proprietor: Gyongyossy-Issa, Maria I. C., Vancouver, BC V6K 3V3 (CA)
(72) Inventor: Gyongyossy-Issa, Maria I.C., Vancouver British Columbia V6K 3V3 (CA); Kizhakkedathu, Jayachandran N., Vancouver British Columbia V6T 1Z4 (CA); Constantinescu, Iren, Vancouver British Columbia V6P 6Y1 (CA)
(74) Representative: Engelhard, Markus

(56) References cited:
- EP-A- 0 160 266
- WO-A-00/28972
- WO-A-01/60339
- WO-A-03/075977
- US-A- 5 049 389
- US-A- 5 534 259
- GIL E S ET AL: "Stimuli-reponsive polymers and their bioconjugates" PROGRESS IN POLYMER SCIENCE, PERGAMON PRESS, OXFORD, GB, vol. 29, no. 12, December 2004 (2004-12), pages 1173-1222, XP004638224 ISSN: 0079-6700
- TORCHILIN V P ET AL: "Poly(ethylene glycol) on the liposome surface: on the mechanism of polymer-coated liposome longevity", BIOCHIMICA ET BIOPHYSICA ACTA. BIOMEMBRANES, AMSTERDAM, NL LNKD- DOI:10.1016/0005-2736(94)90003-5, vol. 1195, no. 1, 12 October 1994 (1994-10-12), pages 11-20, XP023354268, ISSN: 0005-2736 [retrieved on 1994-10-12]
- TORCHILIN V P: "REVIEW POLYMER-COATED LONG-CIRCULATING MICROPARTICULATE PHARMACEUTICALS", JOURNAL OF MICROENCAPSULATION, TAYLOR AND FRANCIS, BASINGSTOKE, GB, vol. 15, no. 1, 1 January 1998 (1998-01-01), pages 1-19, XP000771645, ISSN: 0265-2048
- MOLINEUX G: "PEGYLATION: ENGINEERING IMPROVED PHARMACEUTICALS FOR ENHANCED THERAPY", CANCER TREATMENT REVIEWS, SAUNDERS, US, vol. 28, no. SUPPL. A, 1 April 2002 (2002-04-01), pages 13-16, XP009036301, ISSN: 0305-7372

## Description

### FIELD OF THE INVENTION

The present invention relates to surface-modified lipidic particles and the like, and more particularly, to surface cross-linked lipidic particles useful as pharmaceutical delivery vehicles for biological materials or as artificial platelets and antithrombotics..

### BACKGROUND OF THE INVENTION

Liposomes are small spherical particles formed by lipid bilayers, typically ranging from about 30 nm to 1000 nm in diameter, and serve as convenient delivery vehicles for biologically active compounds, such as small drug molecules, proteins, nucleotides and plasmids.

The field of liposome research has expanded considerably over the last 30 years. It is now possible to engineer a wide range of liposomes and other vesicles varying in size, phospholipid composition and surface characteristics to suit the specific application for which they are intended. For instance, aqueous contrast enhancing agents entrapped in liposomal carriers can be targeted to potential tumour sites for distinguishing between normal and tumour tissue. Topical application of liposome-entrapped drugs has potential for dermatological applications. Liposomes have been used to deliver anticancer agents in order to reduce the toxic effects of the drugs when given alone, or to increase drug circulation time and effectiveness. Liposome-encapsulated hemoglobin (LEH) has been shown to be useful as an oxygen-carrying fluid, capable of surviving for reasonable periods in the circulation. Liposomes may also be used to target specific cells by attaching amino acid sequences, such as antibodies or proteins, or other appropriate materials that target specific receptor sites. Liposomes are also effective as DNA delivery vectors, and are showing potential in DNA vaccination and gene therapy applications.

Conventional liposomes are, however, generally limited by their propensity for fusion with cells, especially with those of circulating blood (Constantinescu et al., 2003, Artificial Cells, Blood Substitutes and Biotechnology 31:394-424). While this property can be exploited to passively deliver drugs to capillary beds located, for instance, in the liver (Koning et al., 2001, Pharm. Res. 18:1291-1298), spleen (Laverman et al., 2000, J. Pharmacol. Exp. Ther. 293:996-1001), or in tumors (Poste, 1983, Biol. Cell. 47:19-38; Mordon et al., 2001, Microvascular Research 63:315-325), targeting liposomes with any specificity remains difficult. Long-term survival of liposomes in the bloodstream is more likely to be due to their association with the blood cells than as individually circulating entities (Constantinescu et al., 2003, *supra;* Mordon et al., 2001, *supra).* Being transported by blood cells is probably how liposomes become localized to areas of rich vascularization (Constantinescu et al., 2003, *supra;* Poste, 1983, *supra;* Mordon et al., 2001, *supra;* Davis et al., 1985, Drugs Under Experimental & Clinical Research 11:633-640).

One approach pursued to enhance the longevity of liposome circulation is through the incorporation of polyethylene glycol (PEG)-bound lipids into the liposome bilayer, for instance as disclosed in U.S. Patents Nos. 6,586,002, 5,395,619, 5,356,633, 5,225,212, 5,213,804, 5,013,556 and U.S. Patent Application Publication No. 2003/0215490. Such liposomes, which have PEG moieties distributed across the liposomal surface, are commonly known as 'sterically stabilized' or STEALTH™ liposomes. These have been used as a vehicle for the delivery of the anticancer agent doxorubicin (Doxil™), although the circulation time of these liposomes *in vivo* is still too short for many other medical applications.

Alternate lipid-based drug delivery systems, such as the coated particle composition disclosed by Zalipsky et al. in U.S. Patent No. 5,534,259, have been developed using particles formed by cross-linked arrays of amphipathic polymer compounds, which comprise a cross-linking region interposed between hydrophilic (PEG) and hydrophobic (lipid) moieties. The cross-linking groups which link adjacent polymer compounds at the linker region form the surface of the particle, thus defining the particle pore size. This approach is therefore limited by the inherent pore size restrictions caused by the relatively short cross-linker molecules.

Using non-fusible materials, such as cross-linked albumin beads or latex spheres, to deliver bioactive material may seem more feasible than using inherently fusogenic liposomes, but such materials are cleared from the circulation relatively rapidly (Lee et al., 2001, Brit. J. Haematol. 114:496-505). While such solid particles do not seem to have a tendency to fuse with blood cells, allowing them to be targeted using a variety of adhesive molecules (Takeoka et al., 2003, Biochemical & Biophysical Research Comunications 312:773-779; Teramura et al., 2003, Biochemical & Biophysical Research Comunications 306:256-260; Davies et al., 2002, Platelets 13:197-205), they are relatively solid objects lacking the aqueous core and lipid bilayer of liposomes, thus limiting their usefulness for hydrophilic or hydrophobic drug delivery.

Another approach to timed drug release, usually dependent on biocompatible matrix degradation, relies on the use of hydrogels based on polymerized macromolecules such as PEGs, acrylates or related block copolymers (U.S. Patent No. 6,911,216; U.S. Patent No. 6,911,227). These hydrogels can be synthesized on a tissue, graft or implant surface for protection or they can be dispersed as small emulsified particles suitable for injection and subsequent blood delivery, once again, to capillary beds. A further variation on this theme is the use of hydrogels with entrapped liposomes containing the desired therapeutic agent dispersed throughout (U.S. Patent No. 5,494,682; U.S. Patent No. 6,056,922.; Uner et al., 2005, Pharmazie. 60:751-755; Ruel-Gariepy et al., 2002. J. Controlled Release 82:373-383). In this case, the benefit derived from the hydrogel is the local retention of the liposomes that are expected to release and deliver the clinically relevant molecules. Hydrogel-liposome dispersions do not, however, allow for free circulation of the liposomes in the bloodstream and are thus limited in their potential applications.

Considering the above-discussed limitations, there is a clear need for a lipidic particle-based delivery vehicle with improved blood circulation properties. For instance, longer-circulating liposomes, micelles or vesicles would be particularly useful in medicinal applications, e.g. as delivery vehicles for encapsulated or surface exposed drugs, dyes or other biological molecules, and could further be used as a platform for the development of artificial platelets.

### SUMMARY OF THE INVENTION

An object of the invention is thus to provide a lipidic delivery vehicle having enhanced stability and increased blood circulation time.

It is also an object of the invention to provide a pharmaceutical composition for use in the delivery of a drug or other medicinally important compound, the pharmaceutical composition facilitating a more controlled release of the compound and/or targeting of the compound to a biological site of interest *in vivo.*
The present invention provides for a lipidic particle, a pharmaceutical composition comprising lipidic particles and to a method for producing a composition of lipidic particles as outlined in the appended claims

Accordingly, as an aspect of the invention, there is provided a method for producing a composition of lipidic particles coated with a cross-linked surface mesh, the method comprising the steps of: (i) preparing lipidic particles comprising pharmaceutically acceptable lipids, (ii) binding hydrophilic polymer chains to the surface of the lipidic particles, and (iii) cross-linking the hydrophilic polymer chains to form the cross-linked surface mesh.

As another aspect of the invention, there is provided a pharmaceutical composition comprising lipidic particles coated with a cross-linked surface mesh; the surface modified lipidic particles comprising: an inner lipidic particle of pharmaceutically acceptable particle-forming lipids; hydrophilic polymer chains linked to the surface of the lipidic particle, the hydrophilic polymer chains comprising a crosslinkable end group at free ends thereof; and cross-linker groups linking the end groups of the hydrophilic polymer chains to form the cross-linked surface mesh.

As a further aspect of the invention, there is provided a lipidic particle surface modified with a cross-linked surface mesh; the surface modified lipidic particle comprising: an inner lipidic particle of pharmaceutically acceptable particle-forming lipids; hydrophilic polymer chains linked to the surface of the lipidic particle, the hydrophilic polymer chains comprising a crosslinkable end group at free ends thereof; and cross-linker groups linking the end groups of the hydrophilic polymer chains to form the cross-linked surface mesh.

The lipidic particles may be combined in a conventional manner with any physiologically acceptable vehicle or carrier including suitable excipients, binders, preservatives, stabilizers, flavours, etc., as accepted in the pharmaceutical practice and appropriate for the intended route of administration.

The lipidic particles include surface-modified nanoparticles or microparticles prepared using varying formulations of pharmaceutically acceptable lipids, and optionally other pharmaceutically acceptable molecules known to assemble into lipidic particles. Examples of the nanoparticles and microparticles include liposomes, lipid or lipid-protein vesicles and micelles.

Formulations of the lipidic particles may be prepared using any pharmaceutically acceptable lipid or related molecule that can form a liposome, lipid or lipid-protein vesicle, or micelle, provided that at least a portion of the lipids and/or related molecules comprise head groups with functionalities that can be chemically modified under mild conditions, i.e., in aqueous conditions, preferably below approximately 50°C. Phospholipids having a free reactive functionality in their head group, such as the free amino group of 1,2-diacyl-sn-glycero-3-phosphoethanolamine (DXPE), are useful in this capacity. The acyl groups, or X, are acyl chains having between 12 to 18 carbons, such as lauryl (L), myristoyl (M), palmitoyl (P), stearoyl (S), arachidoyl (A), oleoyl (O) and combinations thereof. Lipids without a free reactive functionality in their head group may also be included in the formulation. In an embodiment, 1,2 diacyl-sn-glycero-3-phosphocholine (DXPC), whereby the acyl groups are as defined above, is used. In a further embodiment, a mixture of 1,2 dipalmitoyl-sn-glycero-3-phosphoethanolamine (DPPE) and 1,2 dipalmitoyl-sn-glycero-3-phosphocholine (DPPC) is used for preparing the lipidic particles of the present invention. Any other pharmaceutically acceptable lipids, sterols, sphingolipids, detergents, proteins, peptides or hydrophobic, micelle-forming molecules which can be taken up in liposomes, lipid or lipid-protein vesicles, or micelles, may also be included in the formulation. For instance, cholesterol (CHOL) may be included in the formulation as an example of a pharmaceutically acceptable sterol. Oxysterols, such as 15-oxygenated sterols, can also be included in the formulation.

The lipids and/or related molecules comprising head groups with functionalities that can be chemically modified are preferably formulated with the other lipidic particle constituents in a molar ratio of approximately 5 - 95 mol percent of the reactive constituent, more preferably 5 - 40 mol percent, with the remainder made up of the other pharmaceutically acceptable lipids, sterols, sphingolipids, detergents, proteins, peptides or hydrophobic, micelle-forming molecules. Depending upon the nature of the sterol incorporated in the formulation, it is frequently preferable to maintain the molar ratio of the sterol below about 50% of the total formulation molar ratio to facilitate particle extrusion. More preferably, the sterol concentration will be about 40% or lower of the total formulation molar ratio. In an embodiment, the lipidic particles comprise liposomes formulated with a molar ratio of about 40:30:30, respectively, of 1,2-dipalmitoyl-sn glycero-3-phosphoethanolamine, 1,2-dipalmitoyl-sn-glycero-3-phosphocholine, and cholesterol.

The hydrophilic polymer chains are straight chain non-toxic polymers, such as polyethylene glycol (PEG), polyvinylpyrrolidone (PVP), N-substituted polyacrylamides and hydroxyethylacrylates, the polymers comprising a crosslinkable end group such as acrylate, methacrylate, acrylamide and/or methacrylamide. In a preferred embodiment, the hydrophilic polymer is PEG-acrylate.

The length of the polymer chain may vary depending on the nature of the polymer and the lipidic particle of interest. In embodiments incorporating PEG as the hydrophilic polymer chain, the molecular weight (MW) of the PEG may range from 400MW - 20,000MW, preferably between 1,000MW and 10,000 MW, and more preferably about 3400MW with a concentration of the PEG₃₄₀₀ ranging from about 1 mM - 20 mM.

In an embodiment of the PEGylation reaction, the PEG is added in a molar ratio ranging from between about 1:1 lipid:PEG to 4:1 lipid:PEG. A single PEGylation reaction may be conducted, although it is preferred to conduct a plurality of PEGlylation reactions for more efficient PEG loading. In an embodiment, three PEGlylation reactions are conducted.

A cross-linker is used to bridge the free ends of the hydrophilic polymer chains. The cross-linker may include PEG-diacryl, PEG-dimethacryloyl, dimethacrylamide and/or diacrylamide. The length of the cross-linker can be varied depending upon the desired pore size of the surface mesh. For instance, the PEG moiety molecular weight may range from about 700MW - 20,000MW, preferably between 1,000MW - 10,000MW, and will more preferably be approximately 6000MW.

The cross-linking reaction may be conducted using a variety of methodologies known in the art, having regard to the desired cross-linking functionality. For instance, free acrylate groups may be cross-linked in the presence of N,N,N',N'-tetramethylethylenediamine (TEMED) and ammonium or potassium persulfate; or with ammonium persulfate under ultraviolet light, e.g. ultraviolet light at approximately 254 nm wavelength. Alternatively, the cross-linking may be conducted by heating with another water-soluble free-radical initiator at a temperature below the melting temperature of the lipidic particle, e.g. by heating to about 50°C with 2,2'-azobis(2-amidinopropane) dihydrochloride.

The concentrations of the hydrophilic polymer and cross-linkers may range from about 0.5 mM to about 25 mM. Shorter cross-linkers, however, have been found to work better at higher concentrations, for example, diacryl-PEG₇₀₀ is more effective as a cross-linker at concentrations between about 15 mM to 25 mM, more preferably about 20 mM, while diacryl-PEG₆₀₀₀ is effective at concentrations as low as 0.5 mM, more preferably between about 0.5mM - 5 mM, and most preferably about 0.5 mM.

The surface modified lipidic particles of the present invention may be used as a delivery vehicle for encapsulated or lipid-incorporated medicaments or medicinal compounds such as drugs, dyes, recombinant DNA, and biological activity modifying compounds such as energy sources (ATP/ADP), cytokines, hormones and other biological effector molecules. They may also be used for the production of vaccines, due to their long circulating antigen carrier capacity, for the production of antithrombotic materials which interfere with platelet activity, and for the production of artificial platelets.

In an embodiment, the lipidic particles may comprise liposomes or vesicles in which the drug, dye, recombinant DNA or other desired biological material is encapsulated. In this embodiment, the method of the present invention will comprise an encapsulation step in step (i) during the preparation of the lipidic particles.
Encapsulation methods are well known in the art and will vary depending upon the lipids and material to be encapsulated.

Alternatively or additionally, the lipidic particles of the present invention may be further modified at the surface to present ligands, for instance to target the lipidic particles to a site of interest. In such embodiments, the ligands may comprise antibodies, antigens or their representative fragments or epitopes, peptides, drugs that have cell-surface receptors, hormones, biological activity modifiers, enzymes, substrates, potential vaccines, inhibitors and antithrombotic agents.

Use of surface modified lipidic particles as defined above, for *in vivo* delivery of a drug or medicinally important compound, is also provided in accordance with the present invention.

### BRIEF DESCRIPTION OF THE DRAWINGS

Further features and advantages of the present invention will become apparent from the following detailed description, taken in combination with the appended drawings, in which:

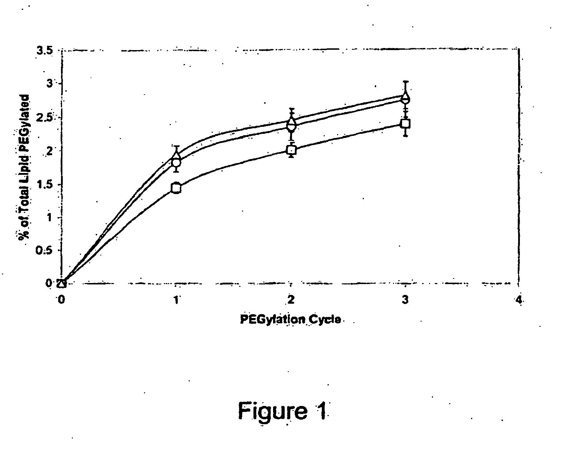

Figure 1 is a plot illustrating that the percentage of lipid PEGylated increases with the amount of DPPE available for PEGylation as well as with the amount of PEG added. Three different levels of DPPE at 20 (squares) 30 (circles) and 40 (triangles) mol% were incorporated into liposomes. Each of these liposomes was subjected to 1, 2 or 3 cycles of PEGylation. The error bars indicate the standard deviations obtained from 3 experiments.

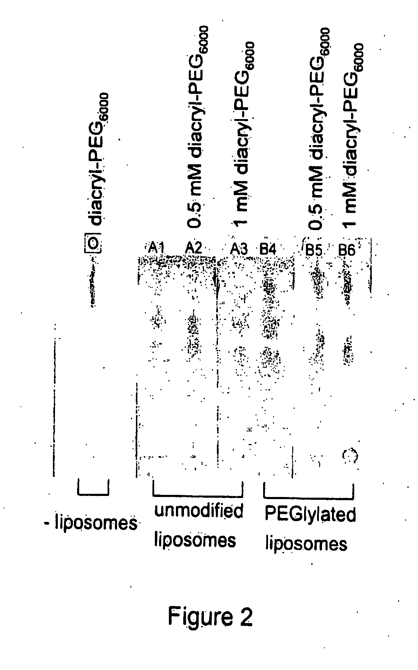

Figure 2 shows the results of thin layer chromatography analysis of untreated and cross-linked liposomes. O: diacryl-PEG₆₀₀₀ and ammonium persulfate, not exposed to UV; A1: unmodified liposomes alone; A2: unmodified liposomes treated with ammonium persulfate and 0.5 mM diacryl-PEG₆₀₀₀; A3: liposomes treated with ammonium persulfate and 1mM diacryl-PEG₆₀₀₀; B4: PEGylated liposomes treated with ammonium persulfate; B5: PEGylated liposomes treated with ammonium persulfate and 0.5 mM diacryl-PEG₆₀₀₀; B6: PEGylated liposomes treated with ammonium persulfate and 1mM diacryl-PEG₆₀₀₀. The TLC was run (bottom to top) on MKC18 reverse phase plates, using a solvent mixture containing chloroform/methanol/water, 40/27/2, by volume.

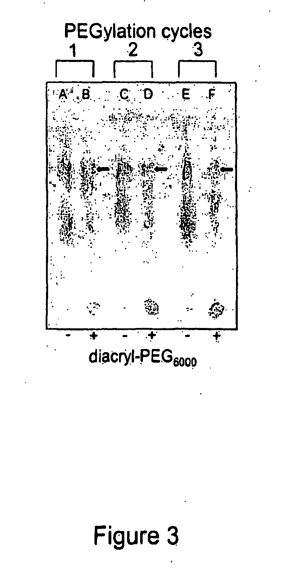

Figure 3 shows the results of thin layer chromatography analysis of liposomes having received 1, 2 or 3 PEGylation cycles and then cross-linked. All the liposomes contained 30 mol% DPPE and received 1 (A&B), 2 (C&D) or 3 (E&F) PEGylation cycles. A, C & E were treated with ammonium persulfate only, while B, D and F also received diacryl-PEG₆₀₀₀. Increasing the number of PEGylation cycles resulted in a corresponding increase of the amount of cross-linked material at the origins of B, D and F, while the DPPE-PEG spots (arrows) decreased compared to its corresponding PEGylation cycle that was not cross-linked. The TLC was run (bottom to top) on MKC18 reverse phase plates, using a solvent mixture containing chloroform/methanol/water, 40/27/2.

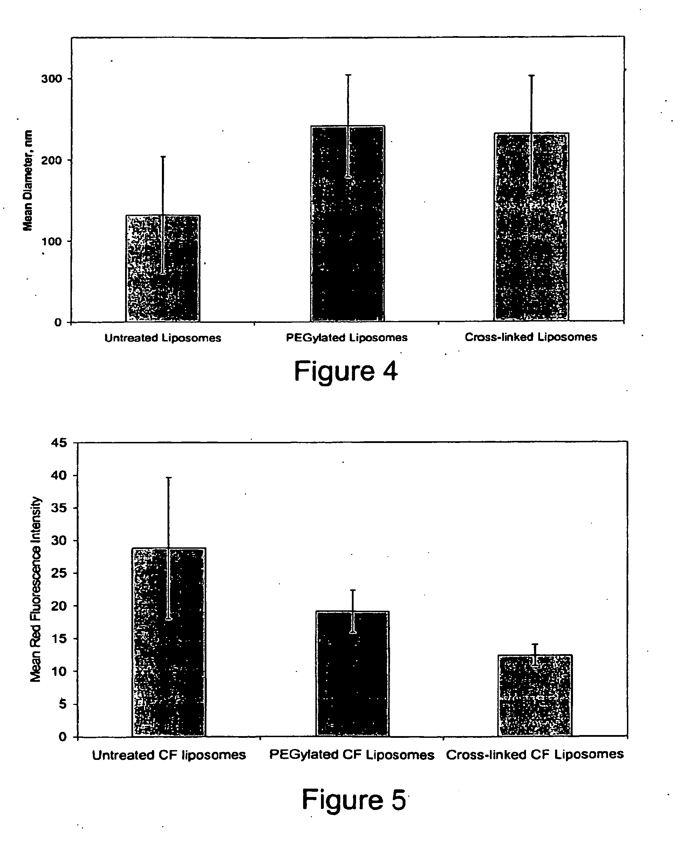

Figure 4 is a plot of liposome mean diameter for untreated, PEGylated and cross-linked liposomes. The Gaussian distribution of the liposomes' mean diameter (nm, y axis) was determined for the untreated, PEGylated and cross-linked liposomes. PEGylation caused an apparent increase of the liposomes' size which was not increased further by cross-linking. The indicated standard deviation for the population is derived by the Nicomp Particle Sizer.

Figure 5 is a plot of the mean red fluorescence intensity of CF-liposomes incubated with the lipophilic fluorophore R18. Untreated, PEGylated, or cross-linked liposomes allowed progressively less incorporation of R18 as measured by the liposomes' fluorescence intensity in the red wavelengths. The bars indicate standard deviation, (n=3).

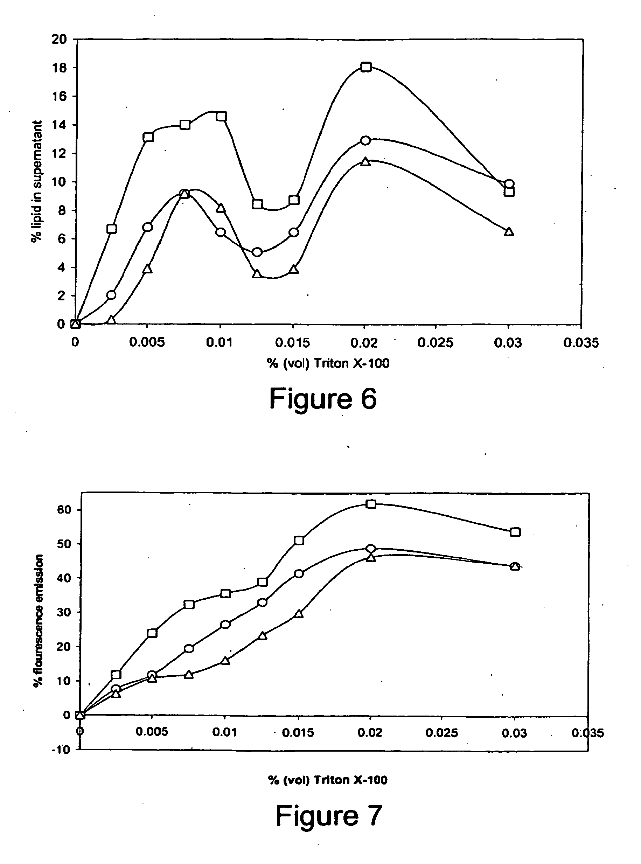

Figure 6 is a plot illustrating the results of lipid extraction from liposomes by Triton™ X-100. The relative amount of lipid found in the liposomes' supernatant is related to the level of protection of the liposome surface afforded by PEGylation and subsequent cross-linking. Untreated liposomes (squares), PEGylated liposomes (circles) and cross-linked liposomes (triangles) were equilibrated with increasing amounts of Triton™ X-100. The 100% lipid level was defined by the lipid concentration of the starting liposome suspension.

Figure 7 is a plot showing the fluorescence emission of EPC-FL-containing liposomes after treatment with Triton™ X-100. Untreated liposomes (squares), PEGylated liposomes (circles) and cross-linked liposomes (triangles) were treated by stepwise addition of Triton™ X-100 from 0 to 1.5% final concentration. The liposomes' supernatant was measured for released headgroup labelled lipid by fluorescence emission at 518 nm. 100% emission was obtained from the liposomes treated with a final concentration of 1.5% Triton™ X-100. The EPC-FL emission level of the liposome suspension before Triton™ X-100 addition was subtracted from all the samples.

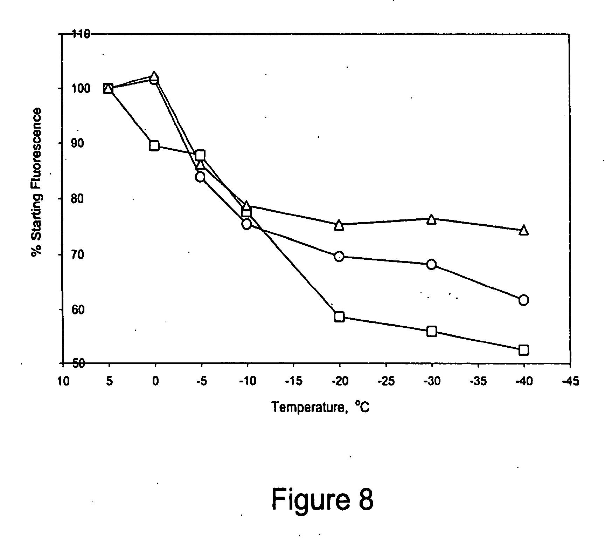

Figure 8 is a plot depicting the effect of cross-linking on liposome cryogenic responses. Untreated (squares), PEGylated (circles) and cross-linked liposomes (triangles) were exposed to controlled rate freezing to the indicated temperatures, followed by rapid thawing. The level of CF fluorescence remaining with the liposome particles was measured by flow cytometry.

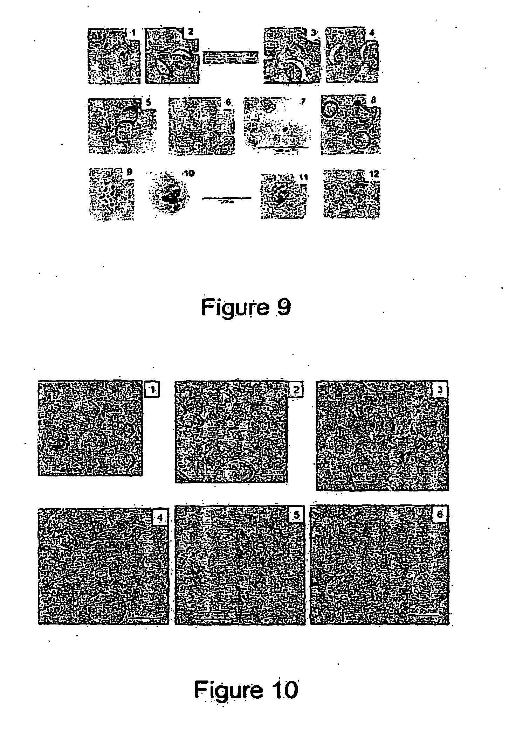

Figure 9 illustrates TEM pictures of unmodified liposomes (1 - 4), PEGylated liposomes (5 - 8) and hydrogel-liposomes (9 - 12). The reference bar is 1000 nm.

Figure 10 illustrates AFM images of dried, unstained liposomes. Unmodified liposomes (1 & 4), PEGylated liposomes (2 & 5) and hydrogel liposomes (3 &6) are shown. The bars represent 200 nm.

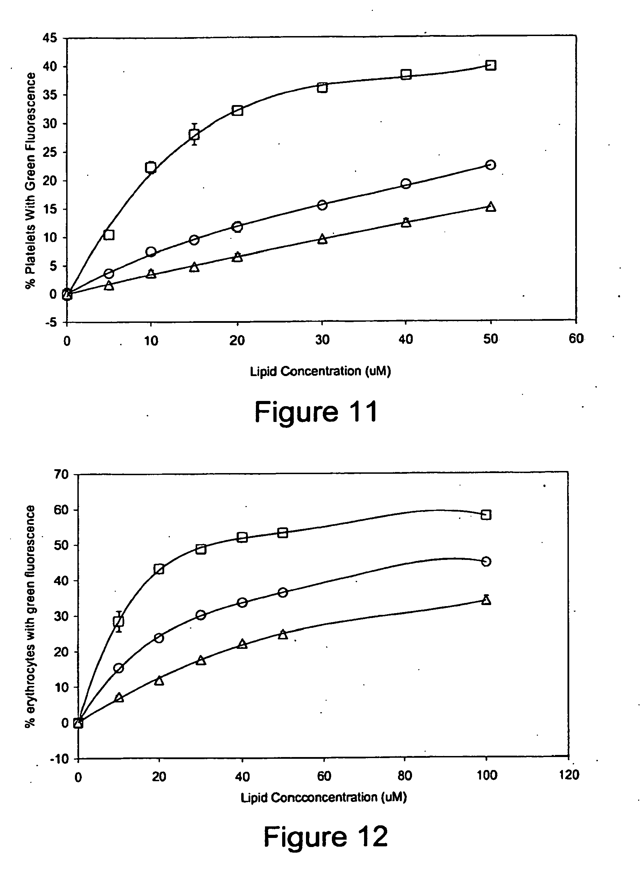

Figure 11 is a plot illustrating the interaction of CF-liposomes with platelets. Increasing concentrations of CF-containing liposomes untreated (squares), PEGylated (circles) or cross-linked (triangles) were allowed to interact with platelets for 2 hours at RT. The platelets were identified with a red fluorescing anti-CD42-PE and the population was assessed by flow cytometry for the proportion of green platelets. The error bars indicate standard deviation.

Figure 12 is a plot showing the interaction of CF-liposomes with erythrocytes. Increasing concentrations of CF-containing liposomes untreated (squares), PEGylated (circles) or cross-linked (triangles) were allowed to interact with red cells for 2 hours at RT. The erythrocytes were identified by their forward and side scatter characteristics in flow cytometry and assessed for the proportion of cells containing the CF marker's green fluorescence. The error bars indicate standard deviation.

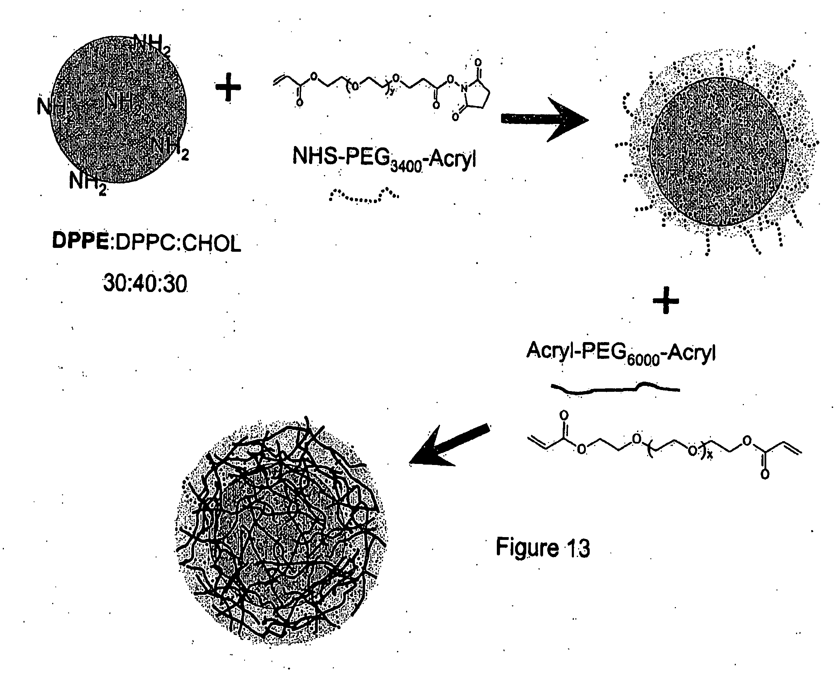

Figure 13 illustrates a scheme of an exemplary route for hydrogel formation on a liposome surface. In the example, hydrogel formation starts with the amines on the lipid phosphatidylethanolamine head group. Acryi-PEG₃₄₀₀-NHS is coupled to these followed by the addition of PEG₆₀₀₀-diacryl to cross-link them, forming the hydrogel.

### DETAILED DESCRIPTION OF THE PREFERRED EMBODIMENT

Disclosed in the following is an exemplary embodiment of a lipidic particle system of the present invention, in which individual liposomes are modified to carry a surface hydrogel layer. The hydrogel is polymerized onto the liposome surface and significantly reduces the liposomes' propensity for fusion with blood cells. At the same time, the liposomes remain as individual units that are not entrapped in a hydrogel matrix, but are generally free to circulate. As both liposomes and hydrogels are eventually biodegradable, these liposomes are particularly suitable for carrying, delivering and slowly releasing hydrophilic drugs from their aqueous core. Furthermore, as the fusibility of these liposomes is greatly reduced, they are suitable for being specifically targeted by biologically relevant molecules that can be attached to the exterior hydrogel layer. Consequently, such hydrogel-carrying liposomes constitute a material that can be used for site-specific delivery and/or controlled release of a drug or other biologically relevant molecule.

### Liposome Preparation

The phospholipids, obtained from Avanti Polar Lipids (Alabaster, AL), were the following: 1,2 dipalmitoyl-sn-glycero-3-phosphoethanolamine (DPPE), 1,2 dipalmitoyl-sn-glycero-3-phosphocholine (DPPC) and L-α-phosphatidyl-N-(Fluorescein) from egg (EPC-FL), while cholesterol (CHOL) was purchased from Sigma-Aldrich (Oakville, ON, Canada). The liposomes used in this study had the following lipid molar ratios: DPPE/DPPC/CHOL 20/50/30; DPPE/DPPC/CHOL 30/40/30 and DPPE/DPPC/CHOL 40/30/30. The lipids were hydrated in buffer containing 280 mM sucrose and 20 mM NaHCO₃ (pH 7.4), with or without 100 µM 5-carboxyfluorescein (CF) purchased from Molecular Probes (Eugene, OR, USA). Some liposomes contained DPPE/DPPC/CHOL/EPC-fluorescein 30/39.7/30/0.3 (molar ratio) and these were hydrated with the same buffer but without the CF marker. The lipids were resuspended in the appropriate buffer by vortexing, then the suspensions were subjected to 5 freeze-thaw cycles using liquid nitrogen, warming to -50 °C and vigorous agitation (Reinish et al., 1988, Thromb. & Haemostas. 60:518-523). The suspensions were maintained at ~50°C and extruded 5 - 10 times through 2 layers of polycarbonate membranes with 400 nm diameter pores (Costar Nuclepore Toronto, ON, Canada), under nitrogen pressure (100-500 lb/in2) using an extruder (Lipex Biomembranes, Vancouver, BC). The resulting liposomes were washed twice with carbonate/bicarbonate buffer, pH 8 (95 mM NaHCO₃, 5 mM Na₂CO₃ and 70 mM NaCI) and centrifuged at 49,000 x g in an Optima TLX Ultracentrifuge (Beckman-Coulter, Mississauga, ON, Canada) to prepare them for the coupling reaction at constant pH, between 7 and 9. The lipid concentration of the liposome suspension was calculated based on a phosphate assay (Fiske et al., 1935, J. Biol. Chem. 66:375-389).

In order to determine the lipid formulation that would maximize PEG derivatization and the relative amount of PEG that becomes coupled to the liposome, three different DPPE concentrations were incorporated into the starting lipid mix to yield 20, 30 or 40 mol-% DPPE. As mentioned above, each of these formulations was subjected to three PEGylation cycles. Data in Figure 1 indicate that the total amount of PEG bound to the liposomes increases with the amount of DPPE present in the liposomes' lipid composition. This is not unexpected, as the available surface amine groups increase and the N-hydroxysuccinimide-ester (NHS) coupling reaction is designed to bind to these primary amine groups. There was only a small increase of the amount of PEG coupled between 30 mol-% and 40 mol-% DPPE. 30% DPPE was thus used as the base formulation for the rest of this study.

### Surface Hydrogel Formation

*PEGylation:* 2 - 3 mL of CF-liposome or EPC-liposome suspensions (20 - 30 mM lipid) in carbonate/bicarbonate buffer were added to dry Acryl-PEG₃₄₀₀-NHS [Shearwater/NEKTAR, Huntsville, AL] at molar ratios ranging from 1:1 lipid:PEG to 4:1 lipid:PEG (in some cases the Acryl-PEG₃₄₀₀-NHS powder was dissolved first in carbonate/bicarbonate buffer and then mixed with the liposomes). After de-gassing with nitrogen for 1 min, followed by 4 hours of incubation and shaking, the liposome/PEG mixture was pelleted for 25 min at 49,000 x g. The free PEG was removed with the supernatant and the pellet was resuspended in fresh buffer (the same volume as removed). The newly PEGylated liposomes were then remixed with the dry Acryl-PEG₃₄₀₀-NHS, using the same procedure, for two more cycles in order to couple more Acryl-PEG₃₄₀₀-NHS to the liposomes' surface. After the third coupling step, the liposomes were washed twice in a bicarbonate buffer containing 150 mM NaCl, 20 mM NaHCO₃ (pH 7.4), and the lipid concentration of the final mixture was determined by the phosphate assay.

The same protocol was done in parallel for unlabeled liposomes (no CF inside, no EPC-FL) to be used as controls. In that case, before each PEGylation step, aliquots (2 x 100 µL) of liposomes were sampled from the bulk liposome batch and added in duplicate, to a homogenous dry mixture of Acryl-PEG₃₄₀₀-NHS / Fluorescein-PEG₅₀₀₀-NHS, 98/2 molar ratio [Shearwater/NEKTAR, Huntsville, AL].

The samples with Fluorescein-PEG₅₀₀₀-NHS were used to quantify (by ratio) the amount of Acryl-PEG₃₄₀₀ that was bound to the liposomes at each step. To reduce the potential for self-quenching by fluorescein (FL), only 2 mol-% fluorescent PEG was used in the mixture. For binding calculations it was assumed that all liposomes coupled under the same conditions were PEGylated at the same rate, resulting in a similar number of PEG molecules attached to the vesicle.

The concentration of FL in the coupled liposome-PEG-FL-2% was detected by fluorimetry on a microplate fluorometer (Spectra Max GeminiXS, Molecular Devices, Sunnyvale, CA) by measuring the emission at 518 nm, (excitation 492 nm) and using a standard curve.

*Cross-linking:* In order to crosslink the liposome-coupled PEG-Acryl, a free monomer that could bridge the acrylate end of the PEG-acrylate was needed. Three different lengths of Diacryl-PEG (700, 3400 and 6000 MW) obtained from SunBio (Anyang City, South Korea) were tested at a range of concentrations, and optimal results were obtained with 1 mM PEG₆₀₀₀-diacryl. The cross-linking reaction was done in bicarbonate buffer using 2 mM (lipid) PEG-liposomes, under UV (Yang et al., 1995, J. Am. Chem. Soc. 117:4843-4850) light at 254 nm (UV Strataliker Crosslinker 1800, Stratagena, LA Jolla, CA) and room temperature (RT), for 100 min using ammonium persulfate as the initiator. The cross-linking reaction was also conducted at room temperature and with natural light but it was found, as by others (Yang et al., 1995, *supra),* that the acrylate-end groups polymerize better under UV light. The cross-linked liposomes were washed twice in bicarbonate buffer and the lipid concentration was measured by the phosphate assay.

### Liposome Characterization

*Demonstration of coupling:* The presence of Acryl-PEG on the liposome surface was confirmed by thin layer chromatography (TLC). TLC was done on MKC18 Silica, 2.5 x 7.5 Whatman plates (Fisher Scientific, Ottawa, ON, Canada) using a solvent mixture containing chloroform/methanol/water, 40/27/2 (by volume) to develop the spots which were visualized by iodine vapour staining.

TLC analysis confirmed the presence of cross-linked PEG on the surface of the liposomes, as the cross-linked material does not migrate with the solvent flow and remains at the origin (Bonte et al., 1987, Biochim. Biophys. Acta. 900:1-9). The TLC analysis further showed that uncoupled lipids move with a retention factor, (Rf) of about 0.64 - 0.72 while the coupled PEG-DPPE moved closer to the solvent front (Table 1), and the native PEG-diacryl₆₀₀₀ (not UV treated) remained at the solvent front (Figure 2).

**Table 1**

| Rf Values | | | | | | | |
|---|---|---|---|---|---|---|---|
| Sample / Lane | 0 | A1 | A2 | A3 | B4 | B5 | B6 |
| PEG-Diacryl₆₀₀₀ | 0.97 | | | | | | |
| Lipids | | 0.72 | 0.72 | 0.71 | 0.70 | 0.66 | 0.64 |
| PEG-DPPE | | | | | 0.89 | | |
| PEG-DPPE & Unreacted PEG-Diacryl₆₀₀₀ | | | | | | 0.99 | 1.00 |
| DPPE-PEG crosslinked | | | | | | 0.03 | 0.04 |

Figure 2 also shows clear differences among the colour intensities of the spots relative to the amount of diacryl-PEG₆₀₀₀ used to crosslink the liposomes: 0.5 mM (Figure 2, B5) and 1 mM (Figure 2, B6). TLC was also used to analyse liposomes that had received three PEGylation cycles using 3 different levels of PEG and were subsequently cross-linked using 1 mM diacryl-PEG₆₀₀₀.

Figure 3 shows the increasing colour intensities of the spots that correspond to cross-linked PEG that remains at the origin. Conversely, analysing the colour intensity of the PEGylated phospholipid spot from these liposomes shows that the intensity of the DPPE-PEG in cross-linked liposomes is less than in the unmodified liposomes, for the same cycle, because some of the DPPE-PEG is retained at the origin with the cross-linked material.

*Liposome* size: Evidence of surface polymer derivatization comes from measurements of the liposomes' mean diameter using quasi-elastic light scattering (Nicomp Submicron Particle Sizer System, Model 370, Santa Barbara, CA, USA). These studies indicate that the liposomes' effective hydrodynamic size increased from ~130 nm to ~230 nm when PEG was coupled to the liposomes. This apparently large increase is more likely related to the initial variation of the liposomes' size as indicated by the wide SD, (also apparent on AFM, vide infra) than the incremental size increase created by the PEG addition. Cross-linking of the acrylate end groups did not cause any further size increases (Figure 4).

### Demonstration of cross-linking:

*(i) Lipophilic fluorophore uptake:* CF-labelled liposomes (200 µL, 1 mM) were incubated for 5 min at RT with 3 µL of a 0.82 mM solution containing the lipophilic marker octadecyl rhodamine B chloride (R18) in ethanol (Molecular Probes, Eugene, OR, USA). After the incubation, the liposomes were diluted up to 2 mL in an aqueous buffer, and analysed by flow cytometry (Beckman Coulter Exel-MCL, Hialeah, FLA). The green liposome bitmap was analysed for red (R18) fluorescence.

By coupling PEG to the liposomes and cross-linking their surface PEG, a network or hydrogel was built around the lipid bilayer that was expected to increase the liposomes' resistance to lipophilic molecules. Figure 5 shows that if a liposome's surface is covered by a strongly hydrophilic layer formed by linear PEG molecules, a lipophilic fluorophore, such as R18, has reduced access to the phospholipid bilayer, resulting in lower red fluorescence. This access is further reduced when the PEG is cross-linked to form a hydrogel.
*(ii) Triton™ X-100 resistance:* Liposomes containing head-group labelled phospholipids EPC-FL (0.33 mM final lipid concentration) were mixed with a range of Triton™ X-100 (Sigma-Aldrich, Oakville, ON, Canada) concentrations (final concentration between 0 % and 1.5 % by volume), incubated for 2 h at room temperature, then centrifuged for 45 min at 21000 x g. The supernatant was analyzed by phosphate assay to quantify the amount of lipid released by the detergent. The amount of EPC-FL released from the liposomes was quantitated by fluorimetry.

Figures 6 and 7, show that PEGylating liposomes, then generating a hydrogel by crosslinking PEG acrylate ends, resulted in increased liposomal stabilility. When liposomes containing headgroup-labelled lipids were mixed with Triton™ X-100 detergent, increasingly more lipid was solubilized from the untreated liposomes, followed by PEGylated, and cross-linked liposomes (Figure 6). Assessment of the release of fluorescent lipids from the three liposome groups paralleled these (Figure 7).
*(iii) Cryogenic responses:* The CF-labelled liposome suspensions were subjected to a controlled-rate freezing and thawing protocol to -40 °C (McGann et al., 1976, Cryobiology 13:261-268). Briefly, 100 µL samples in glass tubes were maintained at 0 °C for 5 minutes in an ice bath, and then placed into a -5 °C alcohol bath (MC880A1, FTS Systems Inc.) for 5 minutes. Extracellular ice formation was induced by touching the outside of the samples with liquid-nitrogen-chilled forceps before the samples were cooled to -40 °C at ~1 °C/min. Samples were removed at 0, -5, -10, -15, - 20, -30, and -40 °C, and rapidly thawed in a circulating 37 °C water bath. The recovered liposomes were analyzed by flow cytometry using a uniform 20 sec. acquisition time and two-colour analysis of the liposome bitmap.

PEGylation and further modification by PEG cross-linking altered the liposomes' cryogenic responses (Figure 8). In this case, liposomes encapsulating CF were subjected to controlled-rate freezing to sub-zero temperatures and rapid thawing, followed by flow cytometric analysis. The total number of liposomes that remained fluorescent remained highest for the cross-linked liposomes (~75 %) with decreasing temperature, as compared to PEG-liposomes (∼60 %), or unmodified liposomes (~50 %). The total number of cross-linked liposomes also remained more stable in terms of size: the slopes of the lines relating final freezing temperature to liposome size were 0.07 for unmodified liposomes, 0.015 for PEGylated liposomes, and 0.0005 for cross-linked liposomes, indicating that unmodified liposomes swelled during thawing, while the PEGylated and cross-linked liposomes resisted swelling the more they were modified (data not shown).
*(iv) Liposome morphology:* Liposomes were visualized by atomic force microscopy (AFM) using a VEECO Digital Instruments (Santa Barbara CA, USA) BIOScope and silicon nitride probes in tapping mode under ambient conditions. Samples were prepared by depositing 10 µL droplets onto freshly cleaved mica, then rapidly dehydrating under vacuum (133 mbar, 30 min). The final lipid concentration was 0.5 mM. Phase images were collected at a scanning rate of 2.5 Hz. Electron microscopy (TEM) was done on a Philips/FEI Tecnai F30 H-7600 electron microscope using negatively stained samples with 2 % uranyl acetate 1 % trehalose (wt/vol) solution.

Both of these methods (AFM and TEM) confirmed that the liposomes remained discrete and that their size distributions were similar to that measured by the Nicomp Particle Sizer. Images 1 - 4 of Figure 9 show that unmodified liposomes have a tendency to collapse during dehydration and staining, which is a well recognized problem in the preparation of liposomes for TEM analysis (Olson et al., 1979, Biochim. Biophys. Acta. 557:9-23). Images 5 - 8 are PEGylated liposomes that have a tendency to trap the stain within the PEG layer, giving a darker outline and a "halo effect." Images 9 - 12 show the hydrogel-liposomes that trap the stain in the surface hydrogel resulting in a "soccer-ball" pattern. These results also indicate that surface-cross-linked liposomes remain stable and resist collapse during dehydration.

AFM is one of the newest techniques employed to image solid lipid nanoparticles (zur Muhlen, A. et al., 1996, Pharm. Res. 13:1411-1416), cells (Radmacher et al., 1992, Science, 257:1900-1905) and liposomes (Anabousi et al., 2005, European Journal of Pharmaceutics and Biopharmaceutics 60:295-303; Ruozi et al., 2005, European Journal of Pharmaceutical Sciences 25:81-89). In "tapping mode, the AFM surface topological images are obtained by gently tapping the surface with an oscillating probe tip. This tool provides visual information, at a nanoscale level, about the size, shape and the surface of the liposomes. However the "halo" and "soccer ball" patterns were not visible due to the samples being unstained. The AFM images also show that PEG crosslinking and the resultant surface hydrogel formation does not lead to liposome fusion, but leave the liposomes as distinct, individual entities (Figure 10: 3). At high magnification, unmodified liposomes appear smooth (Figure 10: 4) while PEGylated liposomes (Figure 10: 5) appear to have a halo. The hydrogel liposomes (Figure 10: 6) appear to be associated with an extra layer of material spreading from the dried liposome.

### Liposome Interaction With Blood Cells

*Blood cells:* Blood samples were obtained from consenting donors as sanctioned by the Research Ethics Boards of both the University of British Columbia and Canadian Blood Services. Blood was drawn into EDTA anticoagulant and used without dilution. Alternately, the various cell types were purified by standard laboratory methods using differential centrifugation (Constantinescu et al., 2003, *supra).* Platelet rich plasma (PRP) was obtained by centrifugation of 5 mL of citrate anticoagulated blood at 200 x g for 15 min (Beckman Coulter GS-6R centrifuge, Hialeah, FLA).

*Interactions:* A range of volumes (0 - 50 µL, containing 1 mM lipid) of internally-labelled CF liposomes (unmodified; PEGylated; and PEGylated-cross-linked) were incubated for 2 hours at room temperature with 5 µL PRP (-100 x 109/L platelets) in 55 µL. Five µL of a specific anti-platelet surface antibody, CD42b (anti-glycoprotein lblX, coupled to phycoerythrin (PE), Beckman Coulter) was added in order to distinguish the platelets from some liposomes that have the same apparent size on the flow cytometer's bitmap. The liposome/platelet/antibody mix was incubated for a further hour at room temperature.

The interaction of red cells (RBC) from whole blood (6 µL) with CF-liposomes (0 - 100 µL, 1 mM lipid) in bicarbonate buffer (200 µL final volume) was also analysed. After a 2.5 h incubation at room temperature, the samples were diluted with 0.8 mL bicarbonate buffer and analyzed by flow cytometry.

Figure 11 shows that, as previously described, platelets take up unmodified liposomes (Constantinescu et al., 2003, supra; Mordon et al., 2001, *supra).* This uptake is much greater than the uptake of cross-linked liposomes and it is dose-dependent.

Figure 12 shows that this is also true of liposome uptake by red blood cells: modified CF-liposomes are taken up to a much lesser extent than unmodified liposomes. Similar results were obtained with liposomes that contained head-group labelled phospholipids (EPC-FL) rather than the encapsulated CF as the fluorescent indicator [data not shown].

### Discussion:

The foregoing experiments demonstrate that it is possible to modify the surface of a lipidic particle, in the present example by creating a hydrogel layer on the surface of a liposome, such that the lipidic particles remain as discrete units and yet acquire new characteristics provided by the surface layer.

In the aforementioned example, the first step to establishing a hydrogel on the liposome surface was to add a PEG layer (Figure 13). A single PEG addition step can be used, although it was observed that a number of low-concentration addition cycles loaded more PEG onto the liposomes and subsequently gave more cross-linked material than a single high concentration step (Figures 1 & 3). As the PEG to be cross-linked was tethered to the liposome via the amino group of a DPPE, leaving only one reactive end free, the effective surface distribution/concentration of PEG also contributed to the hydrogel's formation.

Due to steric/repulsion and solution effects (van Oss, 2003, J. Mol. Recognit. 16: 177-190; Lal et al., 2004, Eur. Phys. J. E15:217-223), the fraction of added PEG that became attached onto the liposome surface decreased with each PEGylation cycle, although only a small proportion of the total available DPPE became substituted (Figure 1). Increasing the mol-% of the liposomes' DPPE to more than 30 mol% did not appreciably increase the amount of attached PEG due to mutual exclusion (van Oss, 2003, supra) by the highly mobile polymer chains (Amsden, 1998, Macromolecules 31:8382-8395; Garbuzenko et al., 2005, Chemistry and Physics of Lipids 135:117-129).

Choosing Diacryl-PEG lengths that resulted in surface gel rather than bulk gel formation was conducted by testing macro monomers of a range of molecular weights. In general, the shorter length Diacryl-PEG chains (e.g. 700 MW) were more difficult to work with in that higher concentrations (about 15 - 25 mM) were required for optimal cross-linking, but at slightly higher concentrations (>25 mM) often resulted in bulk gelation. The optimal concentration range was somewhat wider for Diacryl-PEG 3400 MW. The 6000 MW was easiest to handle, with an optimal concentration range extending as low as 0.5 mM (Figure 2 & Figure 13).

PEGylation increased the effective hydrodynamic diameter of the liposomes compared to those that remained unmodified. However, dynamic light scattering did not show a further size increase after cross-linking (Figure 4). This was also supported by both TEM and AFM analysis (Figures 9 & 10). It was noted that the unmodified sucrose-filled, and therefore more dense, liposomes slowly settled out from the solution, but the PEGylated and cross-linked liposomes remained suspended in the buffer, due to their more hydrophilic surface and the PEG's and the hydrogel's ability to bond to water molecules (Lal et al., 2004, *supra)* while repulsing each other (van Oss et al., 2003, *supra).* Such complex and flexible interactions with the water phase (van Oss 2003, *supra;* Lal et al., 2004, *supra)* may increase the apparent, rather than the calculated actual size of the liposomes (Garbuzenko et al., 2005, *supra).*

The lipophilic fluorophore R18 was used to investigate the establishment of a hydrophilic surface layer on the liposomes. To externally label cells or liposomes, R18 is dissolved in ethanol to carry it through the water phase and into the phospholipid bilayer (Ohki et al., 1998, Biochemistry 37:7496-7503). This caused rapid dye partitioning into exposed phospholipid bilayers (Melikyan et al., 1996, Biophys. J. 71:2680-2691): cells and untreated liposomes took up the dye almost immediately, while PEGylated liposomes took up the fluorophore more slowly. The cross-linked hydrogel was the slowest to take up R18 because the crosslinking restricted R18's diffusional access to the phospholipid bilayer (Figure 5). Diffusion of even relatively small molecules across hydrogels can be restricted by mesh size (Behravesh et al., 2003, Biomaterials, 24:4365-4374). In an end-linked structure, such as the one formed on the liposome surface, the molecular weight between cross-links is the total Diacryl-PEG molecular weight. The relatively short (6000 MW) crosslinking PEG chain lengths, on the ends of the 3400 MW lipid-tethered PEG, define a relatively small mesh size of the order of 1.5 - 3.0 nm (15-30 A); (Stringer et al., 1996, J. Controlled Release 42;195-202; Cruise et al., 1998, Biomaterials 19:1287-1294). The hydrated R18 (732 MW, ~0.55 nm = ~5.5Å) is of the order of magnitude (Baba et al., 2004, J. Chromatography A, 1040:45-51) that can be restricted and its diffusion slowed by such a mesh size (Cruise et al., 1998, *supra).* As well, the relative hydrophobicity of the molecule would alter the ease with which it permeates the channels of moving water among areas of PEG-bound water (Baba et al., 2004, *supra)* of the PEGylated liposome or the fully hydrated hydrogel.

Initially, a similar logic applies to the detergent-based solubilization of the liposomes with Triton™ X-100 (Figures 6, 7). Access of the amphipathic detergent molecules (625 MW) to the phospholipid bilayer would be only slightly faster than the movement of R18, assuming that hydrodynamic diameter is the predominant factor proscribing diffusion. However, in this case, the liposomes were exposed to a range of detergent concentrations. As the detergent solubilized the membrane's constituent phospholipid molecules and removed them from the bilayer, there was consequent formation of incrementally increasing detergent-lipid mixed micelles in the supernatant (Goni et al., 1986, Eur. J. Biochem. 160:659-665). At the critical micellar concentration (CMC) of Triton™ X-100 (0.015 %; 0.2 x 10-3 at 25 °C), the amount of solubilized lipid in the liposomes' supernatant decreased because the detergent-phospholipid mixed micelles were removed by the centrifugation step that removed the liposomes. Overall, more lipid was solubilized from untreated liposomes than from polymer-coated ones. The effects of the detergent were seen at higher detergent concentrations for liposomes with the cross-linked hydrogel, which may be a function of more phospholipid having to be solubilized to create sufficiently large gaps in the lipid-anchored hydrogel and to allow the mixed micelles to escape to the supernatant. The solubilization of fluorescent EPC-FL also shows a similar biphasic curve for untreated liposomes where the saddle point corresponds to the detergent's CMC. PEG and hydrogel-carrying liposomes show incremental increases of lipid-associated fluorescence in the supernatant that reflects not only solubilization of the lipid into a mixed micelle, but also its diffusion out of the hydrated PEG or remaining hydrogel.

The freezing responses of untreated and surface-modified liposomes are perhaps the most interesting. Liposomes are osmotically active vesicles, so like cells, they shrink and swell in response to osmolality changes in their environment (Meryman, 1971, Cryobiology 8:489-500). As the degree of cellular shrinkage has been associated with the extent of freezing damage (Meryman 1971, *supra),* PEGylation, and especially cross-linking, may stabilize liposomes to freeze-thaw by mechanically limiting the degree of shrinkage/expansion that the vesicle can undergo in response to osmotic fluctuations. The hydrogel may also limit the rate of the movement of water across the membrane, as a consequence of the cross-link mesh size and polymer-bound water. This in turn, limits the change of liposome volume that will occur due to the increasing extra-liposomal solute concentration during freezing that would cause the liposomes to shrink. Subsequent to membrane damage by freeze-thaw, membrane breaks would allow the escape of the entrapped CF. However, the PEG, and more so the hydrogel, would either support membrane resealing, or limit the diffusibility of the CF from liposomal aqueous core.

Evidence for the retention of materials in the hydrogel also comes from the TEM images (Figure 9). The uranyl acetate stain used for visualizing liposomes in an electron beam is trapped in the hydrogel layer and produces localized electron dense material that shows up as black spots on the liposome surface. Both these and the AFM images (Figure 10) confirm that the liposomes remain as distinct, regular-sized particles that retain their spherical, cell-like shape and that the cross-linking process does not cause undue fusion or over-all hydrogel formation.

In addition to inhibiting the entry of disruptive molecules and the movement of water, the hydrogel can also prevent lipidic particle fusion with cell membranes. Fusion is thought to take place when membrane proteins have been excluded from the contact region and the phospholipid bilayers form close contacts through local dehydration which is then followed by transient destabilization of the apposed membranes (Bangham et al., 1967, Chemistry and Physics of Lipids 1:225-246; Arnold et al., 1983, Biochim. Biophys. Acta 728:121-128). The PEG molecules' movement is limited due to their mutual repulsion (van Oss, 2003, *supra;* Lal et al., 2004, *supra)* and the hydrogel restricts phospholipid re-ordering by limiting the movement of the hydrogel-tethered phospholipids in the plane of the membrane. The membrane dehydrating tendency of the PEG (Arnold et al., 1983, *supra)* is limited by its attachment to the liposome and to other PEG molecules by cross-linking. Consequently, the coated lipidic particles have a lower tendency to fuse with cell membranes. In the aforementioned example, it is shown that surface modified liposomes fuse with red cells and platelets only to a limited extent (Figures 11,12). At the same lipid:cell ratio, cross-linked liposomes are taken up 3- to 4-fold less than unmodified liposomes, depending on the cell type.

The generation of surface cross-linked lipidic particles, such as liposomes with surface-bound hydrogel, addresses a number of issues encountered during the use of lipidic particles for therapeutic purposes. The reduction of fusion with blood cells is of primary importance: knowing that the material encapsulated in, for instance, a liposome, remains with the liposome and is not transferred, diluted or modified by intracellular enzymes, simplifies the pharmacology of the encapsulated material. As well, drugs that are inadvertently delivered to blood cells by liposomes by liposome-blood cell fusion accumulate in capillary beds. While this is useful for anti-tumour drugs, it may not be appropriate for other therapeutic materials. The coated lipidic particles' ability to carry drugs, either in the aqueous core of a liposome or vesicle, or within the phospholipid layer, makes these lipidic particles superior to cross-linked protein or latex spheres (Takeoka et al., 2003, *supra;* Teramura et al., 2003, supra; Davies et al., 2002, supra) that can only deliver materials covalently attached to them, but which cannot deliver diffusible agents. The cross-linked lipidic particles allow the controlled release of incorporated drugs as the surface layer, e.g. hydrogel, degrades (DuBose et al., 2005, J. Biomed. Materials Research 74A:104-111) and the lipidic particles become accessible to lipases and physiological breakdown (Senior et al., 1984, In: Gregoriadis, G. (Ed.), Liposome Technology, vol. III. CRC Press, Boca Raton, FL, pp. 264-282). Finally, the fact of the cross-linked lipidic particles being suitable for the addition of targeting molecules to their surface solves a major problem: the ability to direct a liposome or other lipidic particle to a specific site. Once at the targeted site, release of the entrapped material will take place as a function of vesicle/liposome stability and surface mesh size and subsequent breakdown (Park et al., 2002, J. Biomedical Materials Research 64A:309-319). Thus, these nanoparticles can be tailor-made by choosing the appropriate polymer chain length, rigidity and crosslinker. Lipidic particles with biocompatible copolymer cross-linked surfaces are thus a stable, biodegradable delivery system suitable for targeted, site-specific drug release at a predetermined rate.

These surface modified lipidic particles have created the possibility of a targeted material that can deliver a drug to the location of choice and deliver it at a predetermined rate, and therefore, represent a new generation of drug carrier systems. They may also be used for the production of vaccines due to their long circulating antigen carrier capacity, for the production of antithrombotic materials which interfere with platelet activity, and for the production of artificial platelets.

## Claims

1. An individual lipidic particle surface modified with a cross-linked surface mesh, the lipidic particle comprising: an inner lipidic particle of pharmaceutically acceptable particle-forming lipids; hydrophilic polymer chains linked to the surface of the lipidic particle, the hydrophilic polymer chains being straight-chain non-toxic comprising crosslinkable end groups at their free ends; and cross-linker groups linking the end groups of the hydrophilic polymer chains to form the cross-linked surface mesh.

2. The lipidic particle according to claim 1, wherein the inner lipidic particles comprise liposomes, vesicles, micelles, or combinations thereof.

3. The lipidic particle according to claim 2, wherein the inner lipidic particles comprise liposomes comprising 1,2 dipalmitoyl-sn-glycero-3-phosphoethanolamine (DPPE), 1,2 dipalmitoyl-sn-glycero-3-phosphocholine (DPPC) and cholesterol (CHOL).

4. The lipidic particle according to any one of claims 1 to 3, wherein the hydrophilic polymer chains comprise polyethylene glycol with an acrylate end group.

5. The lipidic particle according to any one of claims 1 to 4, wherein the cross-linker groups comprise polyethylene glycol diacrylate.

6. The lipidic particle according to claim 5, wherein the polyethylene glycol diacrylate comprises polyethylene glycol with a molecular weight ranging from about 700 to about 20,000.

7. The lipidic particle according to any one of claims 1 to 6, wherein the inner lipidic particles comprise liposomes, vesicles, or combinations thereof and further comprise a drug, dye, recombinant DNA or biological molecule of interest encapsulated therein, and/or antigens or their representative fragments, antibodies, peptides, drugs that have cell surface receptors, hormones, biological activity modifiers, enzymes, substrates, vaccines, potential vaccines, inhibitors, antithrombotic agents, or combinations thereof, bound to the surface thereof.

8. A method for producing a composition of individual lipidic particles with a cross-linked surface mesh according to any of claims 1-7, the method comprising the steps of:
(a) preparing lipidic particles comprising pharmaceutically acceptable lipids,
(b) binding hydrophilic polymer chains to the surface of the lipidic particles, wherein the hydrophilic polymer chains are straight-chain non-toxic polymers comprising crosslinkable end groups at their free ends, and
(c) cross-linking the crosslinkable end groups of the hydrophilic polymer chains to form the cross-linked surface mesh.

9. The method according to claim 8, wherein the lipidic particles in step (a) comprise liposomes, vesicles, micelles, or combinations thereof.

10. The method according to claim 9, wherein the liposomes are prepared using 1,2 dipalmitoyl-sn-glycero-3-phosphoethanolamine (DPPE), 1,2 dipalmitoyl-sn-glycero-3-phosphocholine (DPPC) and cholesterol (CHOL).

11. The method according to any one of claims 8 to 10, wherein the hydrophilic polymer chains in step (b) comprise polyethylene glycol with an acrylate end group.

12. The method according to any one of claims 8 to 11, wherein the cross-linking in step (c) comprises cross-linking the crosslinkable end groups of the hydrophilic polymer chains with a cross-linker.

13. The method according to claim 12, wherein the cross-linker comprises polyethylene glycol diacrylate with a molecular weight ranging from about 700 to about 20,000.

14. The method according to claim 13, wherein the cross-linking is conducted in the presence of ammonium persulfate under ultraviolet light.

15. The method according to claim 12 or 13, wherein the polyethylene glycol diacrylate is diacryl-PEG₇₀₀ at a concentration between about 15 mM and 25 mM.

16. The method according to claim 12 or 13, wherein the polyethylene glycol diacrylate is diacryl-PEG₆₀₀₀ at a concentration between about 0.5 mM and 5 mM.

17. The method according to one of claims 8 to 16, wherein step (a) further comprises encapsulating a drug, dye, recombinant DNA or biological molecule of interest into a liposome or vesicle, and/or binding antigens or their representative fragments, antibodies, peptides, drugs that have cell surface receptors, hormones, biological activity modifiers, enzymes, substrates, vaccines, potential vaccines, inhibitors, antithrombotic agents, or combinations thereof, to the surface of the lipidic particles.

18. A pharmaceutical composition comprising individual lipidic particles with a cross-linked surface mesh according to any of claims 1-7, the discrete lipidic particles comprising:
an inner lipidic particle of pharmaceutically acceptable particle-forming lipids; hydrophilic polymer chains linked to the surface of the lipidic particle, the hydrophilic polymer chains being straight-chain non-toxic comprising crosslinkable end groups at their free ends; and
cross-linker groups linking the crosslinkable free end groups of the hydrophilic polymer chains to form the cross-linked surface mesh.

19. The pharmaceutical composition according to claim 18, wherein the inner lipidic particles comprise liposomes, vesicles, micelles, or combinations thereof.

20. The pharmaceutical composition according to claim 19, wherein the inner lipidic particles comprise liposomes comprising 1,2 dipalmitoyl-sn-glycero-3-phosphoethanolamine (DPPE), 1,2 dipatmitoyl-sn-glycero-3-phosphocholine (DPPC) and cholesterol (CHOL).

21. The pharmaceutical composition according to any one of claims 18 to 20, wherein the hydrophilic polymer chains comprise polyethylene glycol with an acrylate end group.

22. The pharmaceutical composition according to any one of claims 18 to 21, wherein the cross-linker groups comprise polyethylene glycol diacrylate.

23. The pharmaceutical composition according to claim 22, wherein the polyethylene glycol diacrylate comprises polyethylene glycol with a molecular weight ranging from about 700 to about 20,000.

24. The pharmaceutical composition according to any one of claims 18 to 20, wherein the inner lipidic particles comprise liposomes, vesicles, or combinations thereof and further comprise a drug, dye, recombinant DNA or biological molecule of interest encapsulated therein, and/or antigens or their representative fragments, antibodies, peptides, drugs that have cell surface receptors, hormones, biological activity modifiers, enzymes, substrates, vaccines, potential vaccines, inhibitors, antithrombotic agents, or combinations thereof, bound to the surface thereof.

## Patentansprüche

1. Ein einzelnes Lipidteilchen, das mit einem vernetzten Oberflächennetz oberflächenmodifiziert ist, wobei das Lipidteilchen umfasst: ein inneres Lipidteilchen aus pharmazeutisch akzeptablen teilchenbildenden Lipiden; hydrophile Polymerketten, die an die Oberfläche des Lipidteilchens gebunden sind, wobei die hydrophilen Polymerketten geradkettige nicht-toxische Polymere sind, die an ihren freien Enden vernetzbare Endgruppen umfassen; und Vemetzungsgruppen, die die Endgruppen der hydrophilen Polymerketten verbinden, um das vernetzte Oberflächennetz zu bilden.

2. Lipidteilchen nach Anspruch 1, wobei die inneren Lipidteilchen Liposomen, Vesikel, Mizellen oder Kombinationen davon umfassen.

3. Lipidteilchen nach Anspruch 2, wobei die inneren Lipidteilchen Liposomen umfassen, die 1,2 Dipalmitoyl-sn-glycero-3-phosphoethanolamin (DPPE), 1,2 Dipalmitoyl-sn-glycero-3-phosphocholin (DPPC) und Cholesterin (CHOL) umfassen.

4. Lipidteilchen nach einem der Ansprüche 1 bis 3, wobei die hydrophilen Polymerketten Polyethylenglykol mit einer Acrylat-Endgruppe umfassen.

5. Lipidteilchen nach einem der Ansprüche 1 bis 4, wobei die Vemetzungsgruppen Polyethylenglykoldiacrylat umfassen.

6. Lipidteilchen nach Anspruch 5, wobei das Polyethylenglykoldiacrylat Polyethylenglykol mit einem Molekulargewicht im Bereich von etwa 700 bis etwa 20,000 umfasst.

7. Lipidteilchen nach einem der Ansprüche 1 bis 6, wobei die inneren Lipidteilchen Liposomen, Vesikel oder Kombinationen davon umfassen und außerdem, darin eingekapselt, ein Medikament, einen Farbstoff, rekombinante DNA oder ein biologisches Molekül von Interesse und/oder, an ihre Oberfläche gebunden, Antigene oder deren maßgeblichen Fragmente, Antikörper, Peptide, Medikamente mit Zelloberflächenrezeptoren, Hormone, Modifikatoren biologischer Aktivität, Enzyme, Substrate, Impfstoffe, potentielle Impfstoffe, Inhibitoren, antithrombotische Wirkstoffe oder Kombinationen davon umfassen.

8. Ein Verfahren zur Herstellung einer Zusammensetzung einzelner Lipidteilchen mit einem vernetzten Oberflächennetz nach einem der Ansprüche 1 bis 7, wobei das Verfahren die Schritte umfasst:
(a) Bereitstellen von Lipidteilchen, die pharmazeutisch akzeptable Lipide umfassen,
(b) Binden von hydrophilen Polymerketten an die Oberfläche der Lipidteilchen, wobei die hydrophilen Polymerketten geradkettige nicht-toxische Polymere sind, die an ihren freien Enden vernetzbare Endgruppen umfassen, und
(c) Vernetzen der vernetzbaren Endgruppen der hydrophilen Polymerketten, um das vernetzte Oberflächennetz zu bilden.

9. Verfahren nach Anspruch 8, wobei die Lipidteilchen in Schritt (a) Liposomen, Vesikel, Mizellen oder Kombinationen davon umfassen.

10. Verfahren nach Anspruch 9, wobei die Liposomen unter Verwendung von 1,2 Dipalmitoyl-sn-glycero-3-phosphoethanolamin (DPPE), 1,2 Dipalmitoyl-sn-glycero-3-phosphocholin (DPPC) und Cholesterin (CHOL) bereitgestellt werden.

11. Verfahren nach einem der Ansprüche 8 bis 10, wobei die hydrophilen Polymerketten in Schritt (b) Polyethylenglykol mit einer Acrylat-Endgruppe umfassen.

12. Verfahren nach einem der Ansprüche 8 bis 11, wobei die Vernetzung in Schritt (c) die Vernetzung der vernetzbaren Endgruppen der hydrophilen Polymerketten mit einem Vernetzungsmittel umfasst.

13. Verfahren nach Anspruch 12, wobei das Vernetzungsmittel Polyethylenglykoldiacrylat mit einem Molekulargewicht im Bereich von etwa 700 bis etwa 20,000 umfasst.

14. Verfahren nach Anspruch 13, wobei die Vernetzung in Gegenwart von Ammoniumpersulfat unter ultraviolettem Licht durchgeführt wird.

15. Verfahren nach Anspruch 12 oder 13, wobei das Polyethylenglykoldiacrylat Diacryl-PEG₇₀₀ in einer Konzentration zwischen etwa 15 mM und 25 mM ist.

16. Verfahren nach Anspruch 12 oder 13, wobei das Polyethylenglykoldiacrylat Diacryl-PEG₆₀₀ in einer Konzentration zwischen etwa 0,5 mM und 5 mM ist.

17. Verfahren nach einem der Ansprüche 8 bis 16, wobei Schritt (a) außerdem die Einkapselung eines Medikaments, eines Farbstoffs, rekombinanter DNA oder eines biologischen Moleküls von Interesse in ein Liposom oder Vesikel und/oder die Bindung von Antigenen oder deren maßgeblichen Fragmenten, Antikörpern, Peptiden, Medikamenten mit Zelloberflächenrezeptoren, Hormonen, Modifikatoren biologischer Aktivität, Enzymen, Substraten, Impfstoffen, potentiellen Impfstoffen, Inhibitoren, antithrombotischen Wirkstoffen oder Kombinationen davon an die Oberfläche der Lipidteilchen umfasst.

18. Eine pharmazeutische Zusammensetzung, die einzelne Lipidteilchen mit einem vernetzten Oberflächennetz nach einem der Ansprüche 1 bis 7 umfasst, wobei die einzelnen Lipidteilchen umfassen: ein inneres Lipidteilchen aus pharmazeutisch akzeptablen teilchenbildenden Lipiden; hydrophile Polymerketten, die an die Oberfläche des Lipidteilchens gebunden sind, wobei die hydrophilen Polymerketten geradkettige nicht-toxische Polymere sind, die an ihren freien Enden vernetzbare Endgruppen umfassen; und Vernetzungsgruppen, die die vernetzbaren freien Endgruppen der hydrophilen Polymerketten verbinden, um das vernetzte Oberflächennetz zu bilden.

19. Pharmazeutische Zusammensetzung nach Anspruch 18, wobei die inneren Lipidteilchen Liposomen, Vesikel, Mizellen oder Kombinationen davon umfassen.

20. Pharmazeutische Zusammensetzung nach Anspruch 19, wobei die inneren Lipidteilchen Liposomen umfassen, die 1,2 Dipalmitoyl-sn-glycero-3-phosphoethanolamin (DPPE), 1,2 Dipalmitoyl-sn-glycero-3-phosphocholin (DPPC) und Cholesterin (CHOL) umfassen.

21. Pharmazeutische Zusammensetzung nach einem der Ansprüche 18 bis 20, wobei die hydrophilen Polymerketten Polyethylenglykol mit einer Acrylat-Endgruppe umfassen.

22. Pharmazeutische Zusammensetzung nach einem der Ansprüche 18 bis 21, wobei die Vernetzungsgruppen Polyethylenglykoldiacrylat umfassen.

23. Pharmazeutische Zusammensetzung nach Anspruch 22, wobei das Polyethylenglykoldiacrylat Polyethylenglykol mit einem Molekulargewicht im Bereich von etwa 700 bis etwa 20,000 umfasst.

24. Pharmazeutische Zusammensetzung nach einem der Ansprüche 18 bis 20, wobei die inneren Lipidteilchen Liposomen, Vesikel oder Kombinationen davon umfassen und außerdem, darin eingekapselt, ein Medikament, einen Farbstoff, rekombinante DNA oder ein biologisches Molekül von Interesse und/oder, an ihre Oberfläche gebunden, Antigene oder deren maßgeblichen Fragmente, Antikörper, Peptide, Medikamente mit Zelloberflächenrezeptoren, Hormone, Modifikatoren biologischer Aktivität, Enzyme, Substrate, Impfstoffe, potentielle Impfstoffe, Inhibitoren, antithrombotische Wirkstoffe oder Kombinationen davon umfassen.

## Revendications

1. Surface de particule lipidique individuelle modifiée avec un maillage surfacique réticulé, la particule lipidique comprenant : une particule lipidique interne composée de lipides formant particule pharmaceutiquement acceptables ; des chaînes polymériques hydrophiles liées à la surface de la particule lipidique, les chaînes polymères hydrophiles étant linéaires et non toxiques et comprenant des groupes terminaux réticulables à leurs extrémités libres ; et des groupes de réticulation liant les groupes terminaux des chaînes polymères hydrophiles pour former le maillage surfacique réticulé.

2. Particule lipidique selon la revendication 1, dans laquelle les particules lipidiques internes comprennent des liposomes, des vésicules, des micelles ou leurs combinaisons.

3. Particule lipidique selon la revendication 2, dans laquelle les particules lipidiques internes comprennent des liposomes comprenant de la 1,2-dipalmitoyl-sn-glycéro-3-phosphoéthanolamine (DPPE), de la 1,2-dipalmitoyl-sn-glycéro-3-phosphocholine (DPPC) et du cholestérol (CHOL).

4. Particule lipidique selon l'une quelconque des revendications 1 à 3, dans laquelle les chaînes polymères hydrophiles comprennent un polyéthylène glycol ayant un groupe terminal acrylate.

5. Particule lipidique selon l'une quelconque des revendications 1 à 4, dans laquelle les groupes de réticulation comprennent le diacrylate de polyéthylène glycol.

6. Particule lipidique selon la revendication 5, dans laquelle le diacrylate de polyéthylène glycol comprend un polyéthylène glycol ayant un poids moléculaire allant d'environ 700 à environ 20 000.

7. Particule lipidique selon l'une quelconque des revendications 1 à 6, dans laquelle les particules lipidiques internes comprennent des liposomes, des vésicules ou leurs combinaisons et comprennent en outre un médicament, un colorant, un ADN recombiné ou une molécule biologique d'intérêt encapsulé à l'intérieur, et/ou des antigènes ou leurs fragments représentatifs, des anticorps, des peptides, des médicaments comportant des récepteurs de surface cellulaire, des hormones, des agents modifiant une activité biologique, des enzymes, des substrats, des vaccins, des vaccins potentiels, des inhibiteurs, des agents anti-thrombotiques, ou leurs combinaisons, liés à leur surface.

8. Procédé de production d'une composition de particule lipidique individuelle à maillage surfacique réticulé selon l'une quelconque des revendications 1 à 7, le procédé comprenant les étapes suivantes :
(a) la préparation de particules lipidiques comprenant des lipides pharmaceutiquement acceptables,
(b) la liaison de chaînes polymères hydrophiles à la surface des particules lipidiques, les chaînes polymères hydrophiles étant des polymères linéaires non toxiques comprenant des groupes terminaux réticulables à leurs extrémités libres ; et
(c) la réticulation des groupes terminaux réticulables des chaînes polymères hydrophiles pour former le maillage surfacique réticulé.

9. Procédé selon la revendication 8, dans lequel les particules lipidiques de l'étape (a) comprennent des liposomes, des vésicules, des micelles ou leurs combinaisons.

10. Procédé selon la revendication 9, dans lequel les liposomes sont préparés au moyen de 1,2-dipalmitoyl-sn-glycéro-3-phosphoéthanolamine (DPPE), de 1,2-dipalmitoyl-sn-glycéro-3-phosphocholine (DPPC) et de cholestérol (CHOL).

11. Procédé selon l'une quelconque des revendications 8 à 10, dans lequel les chaînes polymères hydrophiles de l'étape (b) comprennent un polyéthylène glycol ayant un groupe terminal acrylate.

12. Procédé selon l'une quelconque des revendications 8 à 11, dans lequel la réticulation dans l'étape (c) comprend la réticulation des groupes terminaux réticulables des chaînes polymères hydrophiles avec un agent de réticulation.

13. Procédé selon la revendication 12, dans lequel l'agent de réticulation comprend le diacrylate de polyéthylène glycol ayant un poids moléculaire allant d'environ 700 à environ 20 000.

14. Procédé selon la revendication 13, dans lequel la réticulation est menée en présence de persulfate d'ammonium sous lumière ultraviolette.

15. Procédé selon la revendication 12 ou 13, dans lequel le diacrylate de polyéthylène glycol est le diacryl-PEG₇₀₀ à une concentration située entre environ 15 mM et 25 mM.

16. Procédé selon la revendication 12 ou 13, dans lequel le diacrylate de polyéthylène glycol est le diacryl-PEG₆₀₀₀ à une concentration située entre environ 0,5 mM et 5 mM.

17. Procédé selon l'une quelconque des revendications 8 à 16, dans lequel l'étape (a) comprend en outre l'encapsulation d'un médicament, d'un colorant, d'un ADN recombiné ou d'une molécule biologique d'intérêt dans un liposome ou une vésicule, et/ou la liaison d'antigènes ou de leurs fragments représentatifs, d'anticorps, de peptides, de médicaments comportant des récepteurs de surface cellulaire, d'hormones, d'agents modifiant une activité biologique, d'enzymes, de substrats, de vaccins, de vaccins potentiels, d'inhibiteurs, d'agents anti-thrombotiques, ou leurs combinaisons, à la surface des particules lipidiques.

18. Composition pharmaceutique comprenant des particules lipidiques individuelles à maillage surfacique réticulé selon l'une quelconque des revendications 1 à 7, les particules lipidiques discrètes comprenant : une particule lipidique interne composée de lipides formant particule pharmaceutiquement acceptables ; des chaînes polymériques hydrophiles liées à la surface de la particule lipidique, les chaînes polymères hydrophiles étant linéaires et non toxiques et comprenant des groupes terminaux réticulables à leurs extrémités libres ; et des groupes de réticulation liant les groupes terminaux libres réticulables des chaînes polymères hydrophiles pour former le maillage surfacique réticulé.

19. Composition pharmaceutique selon la revendication 18, dans laquelle les particules lipidiques internes comprennent des liposomes, des vésicules, des micelles ou leurs combinaisons.

20. Composition pharmaceutique selon la revendication 19, dans laquelle les particules lipidiques internes comprennent des liposomes comprenant de la 1,2-dipalmitoyl-sn-glycéro-3-phospho-éthanolamine (DPPE), de la 1,2-dipalmitoyl-sn-glycéro-3-phosphocholine (DPPC) et du cholestérol (CHOL).

21. Composition pharmaceutique selon l'une quelconque des revendications 18 à 20, dans laquelle les chaînes polymères hydrophiles comprennent un polyéthylène glycol ayant un groupe terminal acrylate.

22. Composition pharmaceutique selon l'une quelconque des revendications 18 à 21, dans laquelle les groupes de réticulation comprennent le diacrylate de polyéthylène glycol.

23. Composition pharmaceutique selon la revendication 22, dans laquelle le diacrylate de polyéthylène glycol comprend un polyéthylène glycol ayant un poids moléculaire allant d'environ 700 à environ 20 000.

24. Composition pharmaceutique selon l'une quelconque des revendications 18 à 20, dans laquelle les particules lipidiques internes comprennent des liposomes, des vésicules ou leurs combinaisons et comprennent en outre un médicament, un colorant, un ADN recombiné ou une molécule biologique d'intérêt encapsulé à l'intérieur, et/ou des antigènes ou leurs fragments représentatifs, des anticorps, des peptides, des médicaments comportant des récepteurs de surface cellulaire, des hormones, des agents modifiant une activité biologique, des enzymes, des substrats, des vaccins, des vaccins potentiels, des inhibiteurs, des agents anti-thrombotiques, ou leurs combinaisons, liés à leur surface.
